(19) Europäisches Patentamt
European Patent Office
Office européen des brevets

(11) Numéro de publication: **0 149 405**
**A2**

(12) # DEMANDE DE BREVET EUROPEEN

(21) Numéro de dépôt: 84402762.3

(22) Date de dépôt: 28.12.84

(51) Int. Cl.4: **G 01 N 33/531**
G 01 N 33/68, G 01 N 33/74
G 01 N 33/82, G 01 N 33/94

(30) Priorité: 30.12.83 FR 8321087

(43) Date de publication de la demande:
24.07.85 Bulletin 85/30

(84) Etats contractants désignés:
AT BE CH DE FR GB IT LI LU NL SE

(71) Demandeur: Etablissement Public dit: CENTRE NATIONAL DE LA RECHERCHE SCIENTIFIQUE (CNRS)
15, Quai Anatole France
F-75007 Paris(FR)

(71) Demandeur: INSTITUT NATIONAL DE LA SANTE ET DE LA RECHERCHE MEDICALE (INSERM)
101, rue de Tolbiac
F-75654 Paris Cedex 13(FR)

(72) Inventeur: Geffard, Michel
23, Avenue du Haut Bron
F-33700 Merignac(FR)

(74) Mandataire: Nony, Michel
Cabinet Nony 29, rue Cambacérès
F-75008 Paris(FR)

(54) **Nouveaux anticorps capables de reconnaitre specifiquement des groupements hapteniques, leur preparation et leur application, et nouveaux antigenes permettant de les preparer.**

(57) Nouveaux anticorps capables de reconnaître spécifiquement des groupements hapténiques, caractérisés par le fait que lesdits groupements hapténiques répondent à la formule :

$$-NH-(CH_2)_n-NH-Hapt.$$

*n* étant un nombre entier pouvant varier de 4 à 6, et Hapt. étant le reste de la molécule d'haptène de formule Hapt.$-NH_2$ ($-NH_2$ étant un groupement amine primaire) ou de formule Hapt. = NH (= NH étant l'imine d'un groupement du type guanidine) ;
leur préparation par immunisation à l'aide d'immunogènes de formule:

$$[M]-[NH-(CH_2)_n-NH-Hapt.]_x ;$$

leur application, notamment au dosage et à la visualisation des haptènes.

EP 0 149 405 A2

Croydon Printing Company Ltd

0149405

Nouveaux anticorps capables de reconnaître spécifiquement des groupements hapténiques, leur préparation et leur application, et nouveaux antigènes permettant de les préparer.

La présente invention a pour objet de nouveaux anticorps capables de reconnaître spécifiquement des groupements hapténiques, leur préparation et leur application, ainsi que de nouveaux antigènes permettant de les préparer.

On sait que de nombreuses molécules de petite taille sont incapables d'induire par elles-mêmes la production d'anticorps, tout en étant capables de réagir avec des anticorps. Ces molécules sont appelées "haptènes". Pour induire la production d'anticorps dirigés contre les haptènes, il est nécessaire de fixer préalablement ceux-ci, généralement de façon covalente, sur une molécule de grande taille appelée "porteur". La substance obtenue (appelée "conjuguée") est alors capable de stimuler la production d'anticorps, et constitue un immunogène.

Par ailleurs, on sait que les réactions antigène-anticorps constituent, en raison de leur grande sensibilité, une méthode particulièrement intéressante pour la détection, le dosage et la visualisation des haptènes, grâce notamment aux techniques d'immunofluorescence et aux dosages radio-immunologiques. Ces méthodes ont déjà été utilisées notamment pour la quantification de diverses hormones.

Dans le domaine de la neurobiologie, on sait que l'identification spécifique des molécules informatives, ainsi que leur localisation dans le système nerveux central, constituent actuellement l'un des sujets de recherche les plus importants, car il permet d'étudier non seulement l'organisation mais aussi le fonctionnement du système nerveux. Il n'est pas douteux que cette étude permettra une meilleure compréhension de divers phénomènes tels que le sommeil, la mémoire, l'olfaction, etc... et permettra aussi de concevoir de nouvelles méthodes de diagnostic et de nouveaux moyens thérapeutiques contre divers troubles du système nerveux. Mais les relations entre les substances neuro-effectrices et la structure du système nerveux central sont très difficiles à étudier et leur étude ne progresse que lentement.

Les méthodes immunologiques apparaissent particulièrement prometteuses dans ce domaine. Toutefois, ici encore, les progrès ont été relativement lents depuis que les premiers essais d'obtention d'anticorps dirigés contre les dérivés indoliques ont été décrits par RANADIVE N. S. et SEHON A.H., Can. J. Biochem. 45, pp. 1689-1700 (1967).

Pour pouvoir mettre en oeuvre ces méthodes immunologiques, le premier problème à résoudre est d'obtenir un immunogène induisant la production d'anticorps hautement spécifiques et affins. On sait que par administration d'un haptène couplé à un porteur (protéine), on obtient en général trois grandes catégories d'anticorps, à savoir ceux qui sont dirigés contre le porteur, ceux qui sont dirigés contre l'haptène et ceux qui sont

dirigés contre un déterminant antigénique situé dans la zone de contact entre l'haptène et le porteur. Généralement, on élimine les anticorps anti-porteur par passage sur un immunoadsorbant solide contenant l'haptène. Seuls les anticorps spécifiques se fixent et peuvent ensuite être élués.

On conçoit donc que la nature du couplage entre l'haptène et le porteur est susceptible de jouer un rôle très important dans l'obtention d'anticorps hautement spécifiques contre l'haptène.

On a proposé récemment le couplage de certaines indolamines par succinylation (M.R. Geffard et al., J. Neurochem. 39, pp. 1271-1277, 1982).

On a maintenant découvert qu'il est possible d'obtenir des anticorps anti-haptène hautement spécifiques à l'aide d'un immunogène obtenu par couplage de l'haptène avec certains dialdéhydes, la réaction de couplage étant suivie d'une réduction des liaisons imine néoformées. Il est généralement possible d'obtenir ainsi des anticorps suffisamment spécifiques pour éviter le passage de l'immunsérum sur un immunoadsorbant contenant l'haptène.

On peut vérifier le degré de spécificité des anticorps obtenus en les testant à l'aide d'antigènes marqués obtenus de la même manière que l'immunogène, par couplage de l'haptène considéré, ou d'haptènes ayant une structure chimique voisine, avec une molécule de faible poids moléculaire ayant un groupement aminé, notamment un dérivé de lysine, à l'aide du même dialdéhyde, le couplage étant éventuellement suivi, comme précédemment, d'une réduction des groupements imine.

La présente invention a pour objet de nouveaux anticorps capables de reconnaître spécifiquement des groupements hapténiques, caractérisés par le fait que lesdits groupements hapténiques répondent à la formule I :

$$-NH-(CH_2)_n-NH-Hapt. \qquad (I)$$

$n$ étant un nombre entier pouvant varier de 4 à 6, et

Hapt. étant le reste de la molécule d'haptène de formule Hapt.-$NH_2$ (-$NH_2$ étant ici un groupement amine primaire) ou de formule Hapt.=NH (=NH étant ici l'imine d'un groupement du type guanidine).

Des exemples d'haptènes particuliers, qu'il est possible de reconnaître grâce auxdits anticorps, sont donnés ci-après à l'occasion de la description du procédé de préparation des anticorps.

L'invention s'étend auxdits anticorps modifiés par marquage avec un traceur radioactif, fluorescent ou enzymatique, obtenus selon les méthodes classiques de préparation de tels anticorps marqués.

Par exemple, le traceur peut être un traceur radioactif obtenu par échange isotopique avec un isotope radioactif tel que I 125. Le couplage des anticorps avec un agent traceur radioactif est connu en soi et peut être réalisé par exemple selon la méthode de GREENWOOD.

Le couplage des anticorps avec un fluorochrome (par exemple

isothiocyanate de fluorescéine ou de rhodamine) est bien connu.

Le couplage des anticorps avec un traceur enzymatique est également connu en soi et peut être réalisé par exemple selon une méthode analogue à celle décrite par NAKANE, Journal Histochemistry Cytochemistry, 22 : 1984-1991 (1974) qui consiste à fixer l'enzyme sur les molécules d'immunoglobulines.

L'enzyme est par exemple une peroxydase ou une phosphatase alcaline.

L'activité enzymatique éventuellement présente sur le réactif, après réalisation d'un test, peut être déterminée selon les méthodes connues avec un substrat approprié permettant par exemple une révélation par colorimétrie, par fluorescence, par luminescence, par potentiométrie, etc ...

L'invention s'étend également aux anticorps monoclonaux obtenus selon la technique connue des hybridomes au départ des lymphocytes prélevés sur les animaux immunisés selon la méthode qui sera décrite ci-après.

L'invention s'étend en outre aux anticorps polyclonaux ou monoclonaux, marqués ou non, fixés sur un support solide permettant leur utilisation comme agents immunoadsorbants dans des méthodes de chromatographie ou comme réactifs d'analyse.

Le support peut être réalisé avec tout matériau solide, biologique ou synthétique, doué de propriétés adsorbantes ou capable de fixer un agent de couplage. Ces matériaux sont connus et décrits dans la littérature. Parmi les matériaux solides capables de fixer les anticorps par adsorption, on citera par exemple le polystyrène, le polypropylène, les latex, etc... Parmi les matériaux utilisables pour fixer les anticorps par covalence à l'aide d'un agent de couplage, on peut citer notamment le dextran, la cellulose, leurs dérivés aminés (diéthylamino-cellulose ou diéthylamino-dextran).

Le support peut se présenter par exemple sous forme de disques, de tubes, de baguettes, de billes, ou de plaques de microtitration.

Les agents de couplage permettant de fixer par covalence les anticorps sur le support solide sont des dérivés bifonctionnels tels que des dialdéhydes, des quinones, etc...

Les anticorps peuvent également être fixés sur des supports solides minéraux selon les méthodes décrites par exemple dans les brevets français n°76.23 176, 77.28 161 et 77.28 163.

L'invention a également pour objet un procédé de préparation des anticorps tels que définis ci-dessus.

Ce procédé est principalement caractérisé par le fait que l'on immunise des animaux par administrations répétées, selon les méthodes connues, d'un ou plusieurs immunogènes de formule II :

$$\left[ M \right] \left[ NH-(CH_2)_n-NH-Hapt. \right]_x \quad (II)$$

M étant le reste d'une macromolécule aminée de formule :

$$( NH =)_{x_2} [ M ] ( -NH_2 )_{x_1}$$

dans lequelle $-NH_2$ est un groupement amine primaire et =NH est l'imine d'un groupement du type guanidine,
Hapt. étant le reste de la molécule d'haptène de formule Hapt.$-NH_2$ ou Hapt=NH, le groupement $-NH_2$ étant un groupement amine primaire et =NH étant l'imine d'un groupement de type guanidine,
n étant un nombre entier pouvant varier de 4 et 6,
$x_1$ étant un nombre égal ou supérieur à 1 et au plus égal au nombre de groupements amine primaire $-NH_2$ portés par ladite macromolécule aminée, et $x_2$ représente zéro ou un nombre au plus égal au nombre de groupements de type guanidine portés par ladite macromolécule aminée, (avec $x_1+x_2 = x$), puis que l'on recueille les anticorps sériques desdits animaux immunisés et que, si désiré, on fixe lesdits anticorps sur un support selon les méthodes connues et/ou les fait réagir avec un marqueur radioactif, fluorescent ou enzymatique selon les méthodes connues. Pour recueillir les anticorps sériques, on utilise les techniques classiques de fractionnement permettant d'isoler les immunoglobulines.

Dans des modes de réalisation préférés, le procédé de préparation des anticorps de l'invention peut encore présenter les caractéristiques suivantes, prises isolément ou en combinaison :

- ledit haptène est un composé choisi parmi des hormones, des acides aminés, des oligopeptides, des vitamines, des drogues, des toxines, des neuromodulateurs et des neuromédiateurs, ainsi que des précurseurs ou analogues de ces composés ;
- ledit haptène est une catécholamine ou un précurseur de catécholamine ;
- ledit haptène est choisi parmi la phénylalanine, la dihydroxyphénylalanine (DOPA), la dopamine, la noradrénaline et l'adrénaline;
- ledit haptène est choisi parmi la tyramine et l'octopamine ;
- ledit haptène est une indolamine ; ladite indolamine est par exemple choisie parmi la sérotonine, le 5-hydroxytryptophane, le 5-méthoxy-tryptophane, la 5-méthoxytryptamine, le tryptophane et la tryptamine ;
- ledit haptène est un acide aminé ou des sels de ces acides ; l'acide aminé est choisi par exemple parmi l'acide glutamique, l'acide aspartique, la taurine et l'acide gamma-aminobutyrique ;
- ledit haptène est un oligopeptide comportant 2 à 5 motifs d'acides aminés ;

- ledit haptène est la tétrodotoxine, la saxitoxine, ou leurs analogues fonctionnels dérivés de la 2-imino hexahydropyrimidine ;

- ladite macromolécule aminée a un poids moléculaire supérieur à 1000 ;

- ladite macromolécule aminée contient des motifs de lysine à groupement $\varepsilon-NH_2$ libre ;

- généralement, on utilise une macromolécule antigénique, bien que cela ne soit pas toujours nécessaire, en particulier lorsqu'il s'agit simplement de faire des injections de rappel lors du processus d'immunisation des animaux ;

- ladite macromolécule aminée est une protéine, un fragment de protéine, ou un polypeptide synthétique ou semi-synthétique (polypeptidyl-protéine), ou encore une paroi de cellules bactériennes dont on a éventuellement éliminé par hydrolyse les déterminants antigéniques polysaccharidiques liés au lipide A, etc... ; on peut citer par exemple une sérumalbumine, une hémoglobine, une polylysine, etc...

L'invention a également pour objet les nouveaux antigènes permettant notamment d'obtenir par immunisation les anticorps décrits ci-dessus.

Ces antigènes constitués par un haptène possédant un groupement amine primaire ou un groupement de type guanidine, lié à une molécule aminée au moyen d'un agent de couplage, sont caractérisés par le fait qu'ils répondent à la formule III :

$$[M'] - [NH-(CH_2)_n-NH-Hapt.]_x \qquad (III)$$

dans laquelle :

M' étant le reste d'une molécule aminée de formule :

$$(HN=)_{x_2}[M'](-NH_2)_{x_3}$$

Hapt. étant le reste de la molécule d'haptène de formule Hapt.-$NH_2$ ou Hapt. = NH, les groupements -$NH_2$ étant des groupements amine primaire et les groupements = NH étant des imines de groupements de type guanidine,

$n$ étant un nombre entier pouvant varier de 4 à 6,

$x_3$ étant un nombre, éventuellement nul, au plus égal au nombre de groupements amine primaire -$NH_2$ portés par ladite molécule aminée, et

$x_2$ étant zéro ou un nombre au plus égal au nombre de groupements de type guanidine portés par ladite molécule aminée, (avec $x_3+x_2 = x$).

L'invention s'étend également auxdits antigènes modifiés par marquage avec un traceur, par exemple un traceur radioactif, ainsi qu'auxdits antigènes fixés sur un support solide selon les techniques classiques de fixation à l'aide par exemple d'agents de couplage bifonctionnels.

Lorsque la molécule aminée est une macromolécule (M' = M) l'antigène est utilisable comme immunogène.

Lorsque la molécule aminée n'est pas une macromolécule, l'antigène peut servir à suivre l'évolution du titre des anticorps lors de l'immunisation et à vérifier la spécificité et l'affinité des anticorps obtenus après immunisation. Comme indiqué ci-dessus, ces antigènes sont préparés de la même façon que les immunogènes mais à partir de molécules aminées $M''-NH_2$ (amine primaire) ou $M'' = NH$ (molécule à groupement de type guanidine) de faible poids moléculaire, généralement inférieur à 1000, et le plus souvent inférieur à 500, comme par exemple le N-$\alpha$-acétyl-L-lysine N-méthylamide ou le tripeptide proline-phénylalanine-lysine ; ces antigènes de formule IV :

$$M''-NH-(CH_2)_n-NH-Hapt. \qquad (IV)$$

font également partie de l'invention. Ils sont généralement utilisés sous la forme d'antigènes marqués avec un traceur, par exemple radioactif, pour étudier les anticorps, selon les méthodes connues, par exemple par la méthode de dialyse à l'équilibre.

L'invention a également pour objet un procédé de préparation d'un antigène tel que défini précédemment.

Ce procédé est principalement caractérisé par le fait :

a) que l'on fait réagir un dialdéhyde de formule :

$$O = CH-(CH_2)_{n-2}-CH = O$$

sur une solution de l'haptène et sur une solution de la molécule aminée,

b) et que l'on soumet le produit obtenu, de formule V :

$$\left[Hapt - Y - (CH_2)_{n-2} - CH = \overset{+}{N}\right]_{x_2}\left[M'\right]\left[N = CH - (CH_2)_{n-2} - Y - Hapt.\right]_{x_3}$$

dans laquelle $x_3$ et $x_2$ sont définis comme précédemment, Y représente un groupement $- N = CH-$ lié par l'azote au reste Hapt lorsque l'haptène a réagi par sa fonction amine primaire, et Y représente un groupement $=\overset{\oplus}{N}=CH-$ lié par l'azote au reste Hapt. lorsque l'haptène a réagi par son groupement de type guanidine,

à l'action d'un agent réducteur capable de réduire les groupements imine ou les groupements $=\overset{\oplus}{N}=CH-$ en groupement amine.

La réaction des aldéhydes sur les amines primaires, ainsi que la réduction des imines obtenues en amines secondaires, sont des réactions bien connues en chimie organique.

De même la réaction des molécules à groupement de type guanidine (ou guanidinium) sur les aldéhydes est connue et procède selon le schéma suivant :

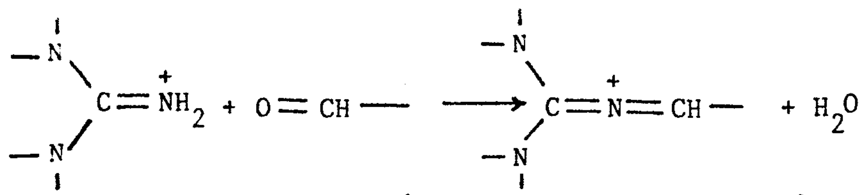

et le composé obtenu est réductible, par exemple par un borohydrure, en composé correspondant de formule

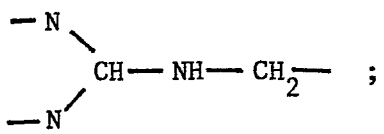

voir par exemple KING, Biochemistry, Vol.5, 3454 (1966) et NAKAYA et al., J. BIOCHEM. TOKYO, 61, 345 (1967).

Dans des modes d'exécution particuliers, le procédé de préparation des antigènes peut encore présenter les caractéristiques suivantes, prises isolément ou en combinaison :

- on fait réagir le dialdéhyde avec l'haptène et avec la molécule aminée ; on opère généralement en solution aqueuse à une température pouvant varier de 15 à 25°C, jusqu'à la fin de réaction de couplage ; la fin de la réaction de couplage est généralement signalée par l'apparition d'une coloration de la solution réactionnelle et par la stabilisation du pH. Le cas échéant, on prend les mesures nécessaires pour éviter l'oxydation de l'haptène, lorsque celui-ci comporte un groupement facilement oxydable ; on peut faire réagir le dialdéhyle en excès soit avec une solution de la molécule aminée, puis on ajoute l'haptène, soit avec l'haptène, puis on ajoute la molécule aminée, soit encore avec une solution contenant à la fois l'haptène et la molécule aminée ;

- Dans le cas où l'haptène est notamment un aminoacide, on fait de préférence réagir l'haptène avec un excès de dialdéhyde (par exemple un excès de 10 fois la quantité stoechiométrique), puis on ajoute la molécule aminée au produit obtenu ; dans le cas d'une molécule aminée à groupements très réactifs, on fait réagir cette molécule d'abord avec un excès de dialdéhyde, puis on élimine le dialdéhyde n'ayant pas réagi (par exemple par dialyse), puis on fait réagir le produit obtenu avec le second réactif aminé et/ou guanidiné ;

- on ajoute le dialdéhyde en quantité suffisante pour faire réagir le maximum de groupements $-NH_2$ et/ou guanidiné de la macromolécule ; l'haptène doit bien entendu être présent en quantités molaires au moins équivalentes ;

- le dialdéhyde est le glutaraldéhyde ;

- l'agent réducteur est un agent non dénaturant dans le cas où le porteur est une protéine ; on utilise en particulier un hydrure et notamment

un borohydrure tel que le borohydrure de sodium.

Bien entendu, si l'haptène comporte des groupements réductibles qui sont réduits en même temps que l'imine, ces groupements peuvent être réoxydés, si désiré, selon les méthodes connues.

L'invention a également pour objet l'application des anticorps tels que définis ci-dessus. Cette application consiste principalement à utiliser les techniques immunochimiques ou immunocytochimiques connues permettant de détecter et de doser dans des solutions, notamment des extraits cellulaires ou tissulaires déprotéinés, ou dans des coupes de tissus convenablement fixés, les haptènes considérés. On peut ainsi étudier la localisation d'un neuromédiateur, le site de fixation d'un médicament, etc...

Par exemple, pour détecter ou doser un haptène en solution, on met en contact la solution à tester avec un support revêtu d'une molécule aminée (par exemple polylysine). Le support est par exemple un support solide utilisable en chromatographie, ou un support constituant un puits de plaque de microtitration. On ajoute un dialdéhyde de formule :

$$O = CH - (CH_2)_{n-2} CH = O$$

(de préférence identique à celui qui a servi à préparer l'immunogène utilisé pour obtenir les anticorps) afin de coupler l'haptène. On peut également utiliser des supports aminés commerciaux sur lesquels est déjà fixé un excès de dialdéhyde, et on met en contact ces supports avec la solution d'haptène. Après réduction des doubles liaisons néoformées, comme précédemment, on met en contact le support avec une solution contenant les anticorps marqués capables de reconnaître sélectivement l'haptène étudié.

Il suffit ensuite de révéler la présence desdits anticorps marqués éventuellement fixés sur le support pour savoir si l'haptène était présent et pour le doser si désiré.

Dans le cas des dosages quantitatifs, la réduction des doubles liaisons amine ou analogues, après couplage ou fixation de l'haptène étudié, est obligatoire.

Dans le cas d'un test qualitatif, en particulier immunocytochimique, cette réduction n'est généralement pas nécessaire, comme cela est montré dans la partie expérimentale.

Autrement dit, généralement, pour les essais immunocytochimiques, on peut se contenter de fixer les coupes de tissus à l'aide du dialdéhyde utilisé dans la préparation des immunogènes ayant servi à obtenir les anticorps, puis on met en contact les coupes de tissus fixés avec les anticorps marqués, dirigés contre l'haptène étudié, dont on révèle ensuite (après rinçage) la fixation éventuelle sur le tissu.

Des indications plus détaillées sur certaines applications spécifi-

0149405

ques seront données ci-après dans la partie expérimentale.

Les exemples suivants illustrent l'invention sans toutefois la limiter.

Dans ces exemples, on désigne parfois les antigènes par des formules abrégées dont la signification est évidente pour les spécialistes. Par exemple, dans le cas où le dialdéhyde est le glutaraldéhyde, on peut désigner le composé de formule V par une formule abrégée, par exemple du type (cas où $x_2 = 0$) :

$$M' \left[ N = G = N - Hapt. \right]_{x_3} \text{ ou } M' \left[ N = G = \overset{+}{N} = Hapt. \right]_{x_3}$$

(ou même par une formule encore plus schématique du type M' = G = Hapt), G étant bien entendu la chaîne carbonée dérivée du glutaraldéhyde, après réaction avec les molécules aminées, c'est-à-dire ici $= CH - (CH_2)_3 - CH =$ . De même, l'antigène de formule III sera noté en abrégé $M' \left[ NH - G - NH - Hapt. \right]_x$, G étant cette fois la chaîne carbonée dérivée du glutaraldéhyde après réduction de l'imine, c'est-à-dire ici $- CH_2 - (CH_2)_3 - CH_2 -$, ou même plus simplement M' - G - Hapt.

Dans certains cas, on a utilisé l'abréviation GA au lieu de G.

**Exemple 1 : Obtention d'anticorps dirigés contre des catécholamines**

**Synthèse des immunogènes à base de dopamine (DA) et de p-tyramine**

Chaque haptène est couplé à la sérum albumine bovine (BSA, Sigma).

On dissout 10 mg de chlorhydrate de dopamine (Sigma) dans 1 ml d'eau distillée. On ajoute 1 ml d'une solution contenant 30 mg de BSA dans 1 ml de tampon acétate 3M, pH 8. On ajoute 1 ml d'une solution de glutaraldéhyde à 5 % (Merck). Après 3 minutes à la température ambiante apparaît une coloration rouge orangée et le pH se stabilise, ce qui marque la fin de la réaction de couplage. On ajoute alors 1 ml d'une solution de 10 mM de borohydrure de sodium (Merck). Le mélange réactionnel devient translucide à la fin de la réaction. On dialyse la solution obtenue contre de l'eau pendant 2 jours à 4°C. Les produits insolubles sont éliminés par centrifugation et filtration à travers un filtre millipore du type 0,45 micromètre. Pour l'analyse, le conjugué obtenu est lyophilisé. Le rapport molaire dopamine/BSA est de 38.

De façon analogue, on a préparé un conjugué de la p-tyramine au départ de 10 mg de chlorhydrate de p-tyramine (Sigma). L'analyse du conjugué lyophilisé montre que le rapport molaire tyramine/BSA est de 40.

**Immunisation**

On immunise des lapins avec les immunogènes obtenus précédemment selon la méthode décrite par Vaitukaitis J.L. et al., J. Clin. Endocr., 33, pp. 988-991 (1971).

Pour chaque immunogène, on administre par voie sous-cutanée à

deux lapins 500 microgrammes d'immunogène émulsionné dans 500 microlitres d'une solution de NaCl 0,15 M et 750 microlitres d'adjuvant complet de Freund. On fait une injection de rappel chaque mois pendant 4 mois. Entre deux rappels, on fait une injection intramusculaire.

Synthèse de ligands radioactifs.

On effectue un couplage de la dopamine (DA) et de la N-α-acétyl-L-lysine N-méthylamide en abrégé (ALM) commercialisée par Sigma, à l'aide de glutaraldéhyde, dans les mêmes conditions que celles décrites ci-dessus pour l'obtention de l'immunogène. Pour cela, on mélange 500 microlitres d'une solution d'ALM 0,1 M pH 8, 100 microlitres d'un tampon acétate 3 M et 40 microlitres de DA tritiée. On ajoute 100 ml d'une solution à 5 % de glutaraldéhyde. Au bout de 3 minutes on ajoute du borohydrure de sodium jusqu'à saturation pendant 30 minutes. On dilue le mélange réactionnel par 1 ml de tampon phosphate et l'on dépose le mélange réactionnel sur une colonne de Séphadex SP C25. On élue avec un gradient de NaCl 10 mM-0,8M dans un tampon phosphate 10 mM de pH 6,2.

De façon analogue, on prépare le conjugué de la tyramine (TA) tritiée et de la N-α-acétyl-L-lysine N-méthylamide.

On prépare aussi de la même façon des conjugués entre la N-α-acétyl-L-lysine N-méthylamide et les composés non radio-actifs suivants : dopamime (DA), tyramine (TA), noradrénaline (NA), octopamine (OA) et L-DOPA.

Incubation

La réaction des ligands ainsi préparés avec les anticorps obtenus ci-dessus est étudiée par la méthode de dialyse à l'équilibre selon les techniques décrites par CAILLA H.L. et al., Anal. Biochem. 56, pp. 383-393 (1973).

Les cellules de dialyse sont séparées par des membranes de cellulose à larges pores (Sartorius S.M. 11 533 ou encore membrane Schleicher et Schull RC 50). On dispose la solution d'anticorps d'un côté de la membrane. On mélange chaque conjugué de catécholamine avec un égal volume de ligand radioactif avant de le placer de l'autre côté de la membrane. La solution d'incubation de chaque antisérum est un tampon Citrate 0,1 M (pH 6,2) contenant 1 mg/ml de sérum albumine bovine (BSA) et 10 mM d'azide de sodium ($N_3Na$). Le volume final est 2 x 150 microlitres. On laisse incuber pendant 20 heures à 4°C. On compte ensuite la β-radioactivité dans un échantillon de 100 microlitres pris de chaque côté de la membrane.

RESULTATS

Pour déterminer, pendant l'immunisation, les titres en anticorps et la réactivité croisée de ces anticorps, on a utilisé la méthode de compétition.

Titres en anticorps anti-DA et anti-TA

Les ligands radioactifs synthétisés ci-dessus sont désignés respectivement par ($^3$H) DA-G-ALM et ($^3$H) TA-G-ALM.

Ces ligands radioactifs on permis de suivre l'évolution de l'immunisation avec une dilution des anticorps au 1/500. Le titre en anticorps avait déjà augmenté au bout de 4 injections (2 rappels et 2 injections intramusculaires).

Pour les anticorps anti-TA, le titre a augmenté fortement après la 5ème injection. Pour ces derniers anticorps, on a pu utiliser une dilution au 1/2000.

Spécificité des anticorps et leur affinité

a) Spécificité des anticorps anti-DA

Pour mener une étude quantitative, on a étudié le déplacement du ($^3$H) DA-G-ALM par la DA non couplée et par les différents dérivés de catécholamines mentionnés ci-dessus. Le meilleur déplacement est observé avec DA-G-ALM, entre $10^{-9}$ et $10^{-7}$ M, ce qui correspond à une constante d'affinité assez élevée (KA = 6,7 x $10^7$ l. mol.$^{-1}$).

La dopamine non modifiée n'est pas capable de déplacer la ($^3$H) DA-G-ALM même à une concentration de $10^{-6}$ M (KA = 6,7 x $10^3$ l. mol.$^{-1}$).

Les dérivés d'autres catécholamines conjuguées avec ALM ont une légère immunoréactivité, très inférieure toutefois à celle de DA-G-ALM. Le composé le plus réactif est NA-G-ALM (KA = 5 x $10^5$ l. mol.$^{-1}$). Ainsi, la réactivité de ce dernier composé est 53 fois moins élevée que celle de DA-G-ALM.

Pour les autres dérivés, la réactivité croisée est encore plus faible. On a trouvé les affinités suivantes :

| Conjugués | KA<br>l. mol.$^{-1}$ |
|---|---|
| L-DOPA-G-ALM | 3,8 x $10^5$ |
| OA-G-ALM | 5 x $10^4$ |
| TA-G-ALM | 3,1 x $10^4$ |

Pour les conjugués OA-G-ALM et TA-G-ALM, on n'a observé aucun déplacement.

b) Spécificité des anticorps anti-TA

On a étudié de la même façon le déplacement de ($^3$H)-TA-G-ALM par les différentes molécules conjuguées appartenant à la famille des catécholamines. Dans cette situation, le meilleur déplacement est observé avec :

TA-G-ALM (KA = 7 x $10^7$ l. mol.$^{-1}$).

La TA non couplée (KA = $10^4$ l. mol.$^{-1}$) est incapable de déplacer le conjugué ($^3H$) TA-G-ALM.

Les conjugués suivants ont montré une faible immunoréactivité :

| Conjugués | KA<br>l. mol.$^{-1}$ |
|---|---|
| OA-G-ALM | 5,9 x $10^5$ |
| L DOPA-G-ALM | 3,3 x $10^5$ |

Pour les conjugués DA-G-ALM (KA = 2,6 $10^4$ l. mol.$^{-1}$) et NA-G-ALM (KA = 1,4 $10^4$ l. mol$^{-1}$) on n'a observé aucun déplacement.

Caractérisation physicochimique des deux antisérums

i) Constantes de dissociation (Kd)

Anti-DA : Kd = 1,48 x $10^{-8}$ M

Anti-TA : Kd = 1,43 x $10^{-8}$ M

Ces constantes ont été obtenues avec le meilleur ligand (DA-G-ALM pour les anticorps anti-DA ; TA-G-ALM pour les anticorps anti-TA)

ii) Il a été possible de calculer la constante d'affinité (KA) pour chaque composé avec le meilleur ligand. Ces valeurs, qui ont été indiquées ci-dessus montrent l'affinité très élevée des anticorps.

iii) L'énergie libre de liaison ($\Delta$Fo = -RT Log KA) a été calculée à 5°C. Les résultats sont les suivants :

Anti-DA $\Delta$Fo = - 9,91 Kcal/mol.

Anti-TA $\Delta$Fo = - 9,93 Kcal/mol.

L'index d'hétérogénéité a été déterminé d'après l'équation de Sips:

$$\log r/2-r = a(\log KA + \log c)$$

où r = B/T ; KA = constante d'affinité ; a = index d'hétérogénéité.

Les résultats sont les suivants :

Anti-DA a = 0,63

Anti-TA a = 0,56

Comparaison de l'affinité des anticorps pour les dérivés non saturés de formule V.

Les anticorps anti-DA et anti-TA reconnaissent respectivement les dérivés non saturés ($^3H$) DA=G=ALM et ($^3H$) TA=G=ALM obtenus par couplage avec le glutaraldéhyde sans réduction ultérieure des groupements imines. Toutefois, les dilutions d'anticorps utilisées étaient beaucoup plus faibles que celles utilisées avec les conjugués saturés (1/200).

L'étude effectuée montre que les courbes de déplacement de ($^3H$) DA=G=ALM par DA=G=ALM et de ($^3H$) TA=G=ALM par TA=G=ALM sont superposées. Pour chaque molécule l'auto-déplacement se situe entre $10^{-8}$ et $10^{-6}$ M.

Les constantes d'affinité ont été calculées pour les ligands non saturés. Les résultats sont les suivants :

Anti-DA KA = $9,1 \times 10^6$ $l.mol.^{-1}$

Anti-TA KA = $6,3 \times 10^6$ $l.mol.^{-1}$.

Ainsi, les anticorps obtenus permettent une reconnaissance des conjugués insaturés. Il n'est donc pas nécessaire, après fixation de l'antigène, d'effectuer la réduction de la double liaison, pour étudier l'antigène à l'aide des anticorps de l'invention.

Il résulte de l'étude précédente des deux anticorps dirigés contre les catécholamines DA et TA qu'une faible différence de structure de l'haptène (présence d'un groupement hydroxyle supplémentaire dans la dopamine) peut induire une réponse immune hautement spécifique pour les deux conjugués différents. Les anticorps anti-DA ne reconnaissent pas le conjugué TA-G-ALM tandis que les anticorps anti-TA n'ont aucune affinité pour le conjugué DA-G-ALM.

Il apparaît donc que les anticorps anti-DA et anti-TA sont utilisables pour l'étude immunocytochimique de la dopamine et de la p-tyramine.

De façon analogue à celle décrite ci-dessus, on a préparé des anticorps anti-NA (NA = nor-adrénaline) et des anticorps anti-OA (OA = octopamine)

<u>Anticorps anti-NA</u>

Les études de spécificité ont montré que le meilleur déplacement est obtenu avec le conjugué NA-G-ALM ; Ka = $3,8.10^7$ l. $mole^{-1}$.

La NA non conjuguée ne provoque pas de déplacement.

<u>Anticorps anti-OA</u>.

Les anticorps anti-OA obtenus sont caractérisés par : Ka = $5.10^6$ $l.mole^{-1}$.

<u>Exemple 2</u> : <u>Obtention d'anticorps dirigés contre des acides aminés.</u>

<u>On décrit ci-après à titre d'exemple le cas de l'acide gamma-aminobutyrique</u>.

<u>Synthèse des conjugués immunogéniques</u>

On mélange 10 mg d'acide gamma-aminobutyrique (GABA) et 2 microlitres de ($^3H$) GABA dans 1 ml d'eau distillée avec 1 ml de tampon acétate 3M de pH 7,8 contenant soit 30 mg de sérumalbumine bovine (BSA, Sigma) ou 30 mg d'hémoglobine bovine (Hb, Sigma) ou 30 mg de poly-L-lysine (PL, Peninsula). On ajoute 1 ml d'une solution à 5 % de glutaraldéhyde. La réaction dure 3 minutes à température ambiante. La fin de la réaction est signalée par l'apparition d'une couleur jaune orangé et par une stabilisation du pH autour de 7. On ajoute alors 1 ml d'une solution 10 mM de borohydrure de sodium (Merck). Après la réduction le mélange réactionnel devient translucide. La solution est alors dialysée à 4°C et le précipité est éliminé

par centrifugation, comme décrit à l'exemple 1.

On compte la $\beta$-radioactivité pour un poids donné de conjugué lyophilisé.

On en déduit que les proportions molaires des conjugués sont les suivantes :

GABA/BSA : 21

GABA/Hb : 50

GABA/PL : 34

Synthèse de ligands radioactifs

De façon analogue à celle décrite à l'exemple 1, on couple le groupement aminé du GABA avec une molécule aminée, qui est ici le tripeptide proline-phénylalanine-lysine (PPL, Bachem), à l'aide de glutaraldéhyde.

La réaction de couplage de GABA tritié désigné par ($^3$H) GABA (Amersham) avec le PPL est effectuée dans les mêmes conditions que le couplage des immunogènes. On mélange 500 microlitres de PPL 0,1 M pH8, 100 microlitres de tampon acétate 3 M, 40 microlitres de ($^3$H) GABA. On ajoute 100 microlitres d'une solution à 5% de glutaraldéhyde. Après 3 minutes on ajoute le borohydrure de sodium et on laisse agir pendant 30 minutes. On dilue le mélange avec 1 ml de tampon phosphate avant de déposer le mélange réactionnel sur une colonne d'éthyl séphadex ammonium quaternaire (QAE Sephadex) (Pharmacia). On élue avec un gradient de chlorure de sodium comme décrit précédemment.

Synthèse de conjugués de divers aminoacides

On effectue de façon analogue à l'aide de glutaraldéhyde un couplage du PPL avec les aminoacides suivants : GABA, glycine, bêta-alanine, glutamate, aspartate, taurine. Après chromatographie sur QAE Séphadex, on calcule la concentration de chaque dérivé par analyse spectrale et comptage de la radioactivité bêta. La proportion molaire de l'aminoacide et du porteur (PPL) est calculée et les concentrations des divers conjugués sont déterminées.

Dans le cas de l'immunogène couplé à la taurine, le groupement sulfonate de la taurine est réduit en même temps que l'imine, mais est facilement réoxydé par l'eau oxygénée ($H_2O_2$) en milieu acétique 0,1 M.

Immunisation

L'immunisation des lapins est effectuée comme décrit à l'exemple 1. Pour la première injection, on utilise comme immunogène le conjugué GABA-GA-BSA ; pour la seconde immunisation, on utilise le conjugué GABA-GA-Hb ; pour la troisième immunisation, on utilise le conjugué GABA-GA-PL ; pour la quatrième immunisation, on utilise le conjugué GABA-GA-BSA, et ainsi de suite.

L'immunisation est effectuée sur une période de 4 mois.

Incubation (essais radioimmunologiques)

Comme à l'exemple 1, on a utilisé la méthode de dialyse à l'équilibre.

Etudes immunocytochimiques

Des cerveaux de rats sont fixés par perfusion avec une solution à 5 % de glutaraldéhyde dans un tampon cacodylate 0,1 M pH 7,5. Le cerveau est découpé en tranches de 4 mm d'épaisseur environ puis fixé à nouveau avec le même fixateur pendant 1,5-2 heures.

Des sections de 50 micromètres sont incubées avec l'antisérum GABA dilué au 1/1500. Cet antisérum est visualisé en utilisant la méthode à la peroxydase-antiperoxydase, avec la diaminobenzidine comme chromogène ; voir Sternberger L.A. IMMUNOCYTOCHEMISTRY (1979) 2ème édition, (John Wiley and Sons, New York) et Buijs R.M. IMMUNOCYTOCHEMISTRY AND ITS APPLICATION IN BRAIN RESEARCH, Van Leeuwen F.W., Swaab D.F., et Buijs R.M. Editeurs (EMBO Amsterdam) pp. 49-55.

Résultats :

Titre de l'antisérum GABA

Le titre maximum est obtenu après la 9ème immunisation, c'est-à-dire au bout de 4 mois.

Spécificité de l'antisérum anti-GABA

Les études de spécificité sont conduites selon la méthode de la dialyse à l'équilibre, par compétition du traceur ($^3H$) GABA-GA-PPL et des différents analogues conjugués GABA-GA-PPL, glycine-GA-PPL, etc...

Le GABA non modifié est incapable de déplacer le ligand radioactif même à une concentration de $10^{-5}$ M.

Les conjugués sont très mal reconnus par l'anticorps ; les composés les plus immunoréactifs sont les conjugués de la bêta-alanine et de la glycine qui ont une réactivité croisée respectivement 175 et 795 fois moindre que le conjugué GABA-GA-PPL.

On n'observe aucune réactivité avec les conjugués d'aspartate et de glutamate, à des concentrations entre $10^{-9}$ M et $10^{-5}$ M. Pour le conjugué de taurine, la réactivité est du même ordre de grandeur qu'avec le conjugué d'aspartate ou de glutamate.

Il faut noter que la purification sur phase solide des antisérums obtenus dans cet exemple n'est pas nécessaire.

Résultats immunocytochimiques

On observe une extinction de la coloration après mélange du sérum natif avec une solution immunogénique. Une diminution très importante de coloration est notée après l'absorption avec une solution $10^{-3}$M de GABA libre.

Aucune différence qualitative ou quantitative de coloration n'apparaît après le mélange du sérum avec les conjugués suivants : glutamate-GA-BSA, aspartate-GA-BSA, taurine-GA-BSA, glycine-GA-BSA et bêta-alanine-GA-BSA.

En utilisant les anticorps anti-GABA sur des coupes fixées au

glutaraldéhyde, on obtient une coloration intense dans les fibres et les cellules de plusieurs régions du cerveau. Plusieurs corps cellulaires réactifs sont visibles même sans utilisation de colchicine. Les fibres contenant du GABA sont trouvées dans pratiquement toutes les régions du cerveau, y compris toutes les couches du cortex cérébral avec plusieurs localisations dans des corps cellulaires, probablement interneurones.

Ces résultats confirment ceux qui ont été observés par d'autres voies. Il faut signaler la présence de GABA en particulier dans l'hippocampe, notamment dans l'innervation des cellules granulaires et pyramidales et, aussi dans les cellules de Golgi et les cellules étoilées du cervelet.

Conclusions

Il est ainsi possible d'obtenir des anticorps contre des petites molécules linéaires telles que le GABA.

La procédure décrite ici permet de produire des anticorps spécifiques très sensibles.

Les immunogènes obtenus après couplage par le glutaraldéhyde sans réduction des doubles liaisons des imines produisent des anticorps qui sont dirigés de façon non spécifique contre lesdites imines, et il est nécessaire d'effectuer plusieurs purifications sur phase solide pour éliminer ces anticorps parasites ; voir à ce sujet Storm-Mathisen J. et al., Nature (London 1983), 301, pp. 517-520.

La réduction des imines formées par réaction avec le glutaraldéhyde apparaît donc comme une étape décisive pour obtenir des anticorps hautement spécifiques.

Les essais de spécificité ont permis de calculer la constante de dissociation et l'énergie libre standard des anticorps préparés.

Ces constantes sont les suivantes :

| Antigène | Kd | $\Delta$ Fo |
|---|---|---|
| GABA-GA-lysine | $2.10^{-8}$M | -9,8 Kcal/mol |

Les études immunocytochimiques effectuées sur des coupes de cerveaux à l'aide des anticorps anti-GABA ont permis de confirmer les découvertes antérieures indiquant que les neurones GABA innervent pratiquement toutes les parties du système nerveux central.

De façon analogue, on a préparé des anticorps dirigés contre les aminoacides suivants :

- taurine
- acide aspartique
- acide glutamique

Pour les anticorps anti-taurine, on a trouvé : Kd = $5.10^{-8}$ M.

Exemple 3 : Préparation d'anticorps dirigés contre des indolamines

De la même façon, on a préparé des anticorps dirigés contre chacune des diverses indolamines suivantes.

Synthèse des immunogènes

En opérant comme dans les exemples précédents, on a préparé les immunogènes suivants :

BSA - G - W

BSA - G - HW

BSA - G - MW

BSA - G - T

BSA - G - MT

BSA - G - HT

ainsi que les mêmes immunogènes avec HSA au lieu de HSA.

Abréviations : BSA : sérum albumine bovine ;
HSA : sérum albumine humaine ; W : L-tryptophane ;
HW : 5-hydroxytriptophane ; MW : 5-méthoxy-tryptophane ;
T : tryptamine ; MT : 5-méthoxytryptamine ; HT : 5-hydroxytryptamine (ou sérotonine)

Synthèse de ligands

Comme dans les exemples précédents, on a préparé les ligands conjugués suivants :

W-G-PL ; HW-G-PL ; MW-G-PL ; HT-G-PL ; MT-G-PL ; T-G-PL.

On a également étudié pour comparaison les conjugués non réduits intermédiaires, soit :

W = G = PL ; HW = G = PL ; MW = G = PL ; HT = G = PL ; MT = G = PL ; T = G = PL.

Abréviation : PL signifie poly-L-lysine (P.M. 60.000)

Immunisation et obtention des anticorps.

On a opéré comme dans les exemples précédents.

Etude de l'affinité et de la spécificité des anticorps.

Cette étude a été effectuée à l'aide d'un test ELISA (Enzyme-linked immunosorbent assay). Les plaques sont revêtues avec une solution de conjugué BSA-G-indolamine puis saturées à l'aide de sérum bovin. On met ensuite en contact avec la solution d'anticorps étudiés, puis avec des anticorps de chèvre anti-(gammablobulines de lapin) conjugués à la peroxydate de raifort. On révèle la fixation des anticorps avec une solution d'o-phénylènediamine contenant de l'eau oxygénée, en mesurant la densité optique.

RESULTATS

Anticorps anti-W :

Les courbes de déplacement obtenues par la méthode de compétition montrent que le meilleur déplacement est observé avec W-G-PL. L'auto-déplacement se situe entre $10^{-9}$M et $10^{-7}$M. Le tryptophane non conjugué n'est que faiblement reconnu. Le conjugué non réduit W = G = PL est 444 fois moins réactif que W - G - PL.

Anticorps anti-HW :

Le meilleur déplacement est obtenu avec HW - G - PL (5 fois moindre avec HW = G = PL).

Anticorps anti-HT :

Le meilleur déplacement est obtenu avec HT - G - PL. L'haptène non conjugué n'est que faiblement reconnu.

Anticorps anti-MW :

Meilleur déplacement avec MW - G - PL.

L'immunoréactivité de MW = G = PL est 13 fois plus faible.

Anticorps anti-MT :

Meilleur déplacement avec MT - G - PL (12 fois moindre avec MT = G = PL).

Les essais de spécificité ont permis de calculer les constantes Kd qui sont indiquées dans le tableau suivant :

| Haptènes | Constante de dissociation Kd (moles/litre) |
|---|---|
| 5-méthoxytryptophane | $1,40 - 10^{-9}$ |
| Tryptophane | $7,2 - 10^{-9}$ |
| 5-hydroxytryptophane | $1,35 - 10^{-8}$ |
| 5-méthoxytryptamine | $2,2 - 10^{-8}$ |
| Tryptamine | $2,4 - 10^{-9}$ |
| Sérotonine | $3,0 - 10^{-9}$ |

Exemple 4 : Préparation d'anticorps anti-tétrodotoxine

On rappelle que la tétrodotoxine(en abrégé : TTX)répond à la formule suivante :

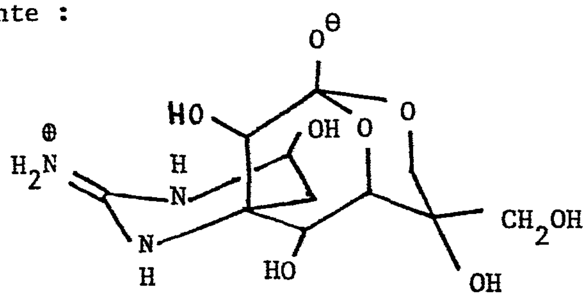

Synthèse de l'immunogène

Elle est effectuée par deux procédés :

A) - Activation préalable de la protéine porteuse par le glutaraldéhyde (G) :

1. - activation de la protéine porteuse par le glutaraldéhyde : à 30 mg de sérum albumine humaine (HSA) dilués dans 1,500 ml de solution d'acétate de sodium 1 M, pH = 8, on ajoute très rapidement 3 ml d'une solution de glutaraldéhyde 1 M (Merck) ; le mélange est dialysé contre de l'eau à 4°C pendant 2 jours pour éliminer le glutaraldéhyde non fixé.

On obtient le produit de couplage HSA = G.

2. - 5 mg de la protéine activée au glutaraldéhyde sont repris après lyophilisation dans une solution d'acétate 0,75 M, pH : 7,5 et mélangés à 300 microlitres d'une solution de TTX à 1 mg/ml dans de l'eau. On laisse le mélange pendant 30 min à la température ambiante. On obtient le conjugué non réduit HSA = G = TTX.

Le pH est ramené à 6 par l'addition de 500 microlitres de tampon-citrate 0,2M, pH = 4,8.

3. Les groupements imine néoformés sont réduits par l'addition d'une solution 0,1 M de borohydrure de sodium.

4. - Le conjugué réduit HSA - G - TTX est dialysé pendant 1 jour 1/2 à 4°C contre de l'eau.

5. - un prélèvement est fait sur chaque dialysat (n = 4) dans le but de tester à l'aide d'un bio-essai (dont la technique est exposée ci-après) la concentration de TTX relarguée au cours de la dialyse (TTX non conjuguée). L'activité biologique de chaque immunogène est testée par cet essai.

Le rapport molaire TTX/albumine du conjugué obtenu est compris entre 3 et 4.

B) - Activation préalable de la TTX par le glutaraldéhyde :

1) - Une solution (1 ml) $10^{-3}$M de TTX en milieu acétate de sodium 2M, pH : 7,6, est activée par 50 microlitres de glutaraldéhyde (G) 0,1M (soit une concentration en G de $5.10^{-3}$M). Le pH final du mélange est de 7,2. On obtient le produit de couplage G = TTX.

2) - Après une 1/2 heure, on ajoute 500 microlitres d'une solution d'acétate de sodium 0,75M contenant 8 mg de sérum albumine (HSA). Le mélange est laissé ainsi une 1/2 heure à la température du laboratoire. La TTX = G se fixe sur les groupements aminés libres de la protéine pour donner le conjugué : HSA = G = TTX.

3) - Les doubles liaisons des imines néoformées sont réduites après addition de 500 microlitres de tampon citrate 0,2M, pH = 4,8 contenant du borohydrure de sodium. On obtient le conjugué HSA - G - TTX.

4) - Le conjugué est dialysé contre l'eau à 4°C pendant 3 heures pour enlever l'excès de TTX non conjuguée ou les dimères TTX - G - TTX facilement dialysables.

La concentration de TTX fixée est appréciée par le bio-essai, en mesurant sur un aliquote l'activité de la TTX à chaque étape de l'hémisynthèse du conjugué immunogénique.

Le rapport molaire TTX/albumine du conjugué obtenu est compris entre 10 et 15.

Immunisation

L'immunisation est effectuée comme dans les exemples précédents.

Etude de la spécificité des anticorps anti-tétrodotoxine par une méthode ELISA

Ce test comprend 4 étapes successives :

1) - Adsorption de l'antigène (ou immunogène) sur la plaque de microtitration (Nunc).

2) - Réaction antigène-anticorps (complexes I).

3) - Formation de complexes II par fixation d'anticorps anti-IgG de lapins couplés à la peroxydase, sur les complexes I.

4) - Visualisation des complexes II par action de la peroxydase sur un substrat chromogène : orthophénylènediamine (OPD).

1ère étape : revêtement de la plaque ou "coating"

Des conjugués TTX - G - HSA (et HSA - G pour comparaison) sont dilués dans du tampon carbonate $5.10^{-2}$M pH = 7,5. La dilution utilisée dans ce système est donnée par le meilleur rapport signal/bruit. Dans ce cas, elle est de 7,5 µg/ml. 200 µl de chaque solution sont déposés dans les puits et laissés pendant 16 heures à 4°C. Après ce temps, chaque puits est vidé et rincé 3 fois avec du tampon A(tampon phosphate $10^{-2}$M, NaCl 0,15M, pH : 7,4).

2ème étape : application de l'anticorps primaire (anticorps anti-TTX)

Au préalable, le sérum est adsorbé sur des protéines traitées au glutaraldéhyde et réduites pendant 16 heures à 4°C.

Les immun -complexes obtenus par reconnaissance des immunoglobulines anti-protéines porteuses sont éliminés par immunoprécipitation. Le sérum prépurifié est dilué au 1/20.000 dans du tampon A auquel on ajoute 1 % de sérum de boeuf. 200 microlitres sont déposés dans chaque puits ayant, soit l'immunogène TTX - G - HSA ,soit G-HSA. Une incubation de 2 heures permet à la réaction antigène-anticorps d'être optimale. Elle est suivie de 3 rinçages avec du tampon A.

3ème étape : application des anticorps anti-IgG marqués à la peroxydase (anticorps secondaires). Les anti-IgG de lapin proviennent de

sérums immuns de chèvre. Elles sont diluées au 1/10.000 dans le tampon A contenant 1 % de sérum de boeuf. La durée d'incubation est 1 heure à 37°C. Elle est suivie par 3 lavages avec du tampon A.

4ème étape : application de l'OPD

200 microlitres d'une solution contenant de l'eau oxygénée ($H_2O_2$) et de l'OPD sont déposés dans chaque puits. La solution est préparée extemporanément dans une solution de tampon citrate-phosphate 0,1M, pH = 5, de façon à obtenir une concentration finale de 0,5 mg/ml d'OPD et 0,025 % d'$H_2O_2$. La réaction évolue pendant 10 minutes à l'obscurité et est arrêtée par l'addition de 50 microlitres d'acide sulfurique 4N dans chaque puits.

L'absorbance est lue à 492 nm au spectrophotomètre.

Les valeurs observées sont corrigées en soustrayant les valeurs expérimentales lues dans les puits témoins contenant G - HSA.

RESULTATS

- Spécificité des anticorps-anti TTX

Par compétition entre l'immunogène TTX - G - HSA déposé dans les puits et le conjugué pré-incubé avec l'anticorps purifié pendant 1 heure à 37°C, on obtient des courbes de déplacement montrant que l'auto-déplacement intervient entre $10^{-10}$ et $10^{-7}$M pour les immunsérums obtenus. Ce résultat indique une bonne affinité des sites anticorps obtenus pour le groupement haptènique TTX - G-. En effet la TTX non conjuguée est moins bien reconnue. Ce résultat souligne l'importance du résidu glutaraldéhyde réduit dans l'immunoreconnaissance.

L'immunogène TTX - G - HSA, synthétisé d'après la première méthode A, conserve la propriété de bloquer le potentiel d'action, cet effet étant comparable, apparemment, à celui de la TTX initiale. La dialyse de l'immunogène permet d'éliminer presque toute trace de TTX libre. Dans ces conditions, l'effet bloquant de l'immunogène est approximativement 50 fois plus fort que l'effet toxique de la TTX libre se trouvant dans le troisième dialysat de l'immunogène TTX - G - HSA. L'immunogène synthétisé par la méthode B est encore plus actif, la TTX - G - HSA, après dialyse, étant approximativement 500 fois plus active que la TTX libre se trouvant dans le dialysat.

## Technique de dosage de la TTX par le bloquage de la conduction de l'influx nerveux

(technique de dosage de la TTX par bioessai)

L'effet de bloquage de la conduction de l'influx nerveux en fonction de la concentration de la Tetrodotoxine (TTX) a été étudié sur le nerf sciatique de la grenouille. La technique utilisée est similaire à celle

décrite par Seeman et coll (Canad. J. Physiol. Pharmacol., 50, 1181-1192 (1972)) et Levinson (Phil. Trans. R. Soc. London B, 270, 337-348 (1975)). Après le prélèvement du nerf sciatique, celui-ci est dégaîné. Cette technique permet un accès direct et rapide de la toxine aux noeuds de Ranvier.

Le nerf dégaîné est placé dans une chambre en plexiglass à 5 compartiments. Les deux compartiments de chaque côté de la chambre contiennent respectivement des électrodes de platine pour stimulation et enregistrement tandis que le compartiment central sert à appliquer la toxine. Les compartiments sont isolés électriquement l'un de l'autre. Les compartiments sont remplis avec la solution Ringer pour grenouille. La chambre est maintenue à température constante (20°C).

Le nerf est stimulé toutes les deux secondes avec un neurostimulateur. Les potentiels d'action sont enregistrés.

La courbe dose-réponse est obtenue en représentant le pourcentage des amplitudes des potentiels d'action non bloqués du nerf en fonction de la concentration de la TTX par rapport à l'amplitude du potentiel d'action du même nerf dans la solution Ringer. La concentration de tétrodotoxine qui induit 50 % de bloquage de l'amplitude du potentiel d'action ($A_{50}$) se situe entre 15 et $30.10^{-9}$M, la précision de la méthode étant de l'ordre de ± 5 %.

La composition de la solution Ringer utilisée, pour 1 litre, est la suivante : NaCl 110mM, KCl 2,5mM, $CaCl_2$ 2mM, Tris 5mM, pH : 7,3.

REVENDICATIONS

1. Nouveaux anticorps capables de reconnaître spécifiquement des groupements hapténiques, caractérisés par le fait que lesdits groupements hapténiques répondent à la formule :

$$-NH-(CH_2)_n-NH-Hapt.$$

n étant un nombre entier pouvant varier de 4 à 6, et Hapt. étant le reste de la molécule d'haptène de formule Hapt.-$NH_2$ (-$NH_2$ étant ici un groupement amine primaire) ou de formule Hapt.=NH (=NH étant ici l'imine d'un groupement du type guanidine).

2. Anticorps selon la revendication 1, caractérisés par le fait que ledit haptène est un composé choisi parmi des hormones, des acides aminés, des oligopeptides, des vitamines, des drogues, des toxines, des neuromodulateurs et des neuromédiateurs, ainsi que des précurseurs ou analogues de ces composés.

3. Anticorps selon la revendication 1, caractérisés par le fait que ledit haptène est une catécholamine ou un précurseur de catécholamine.

4. Anticorps selon la revendication 1, caractérisés par le fait que ledit haptène est choisi parmi la phénylalanine, la dihydroxyphénylalanine (DOPA), la dopamine, la noradrénaline et l'adrénaline.

5. Anticorps selon la revendication 1, caractérisés par le fait que ledit haptène est choisi parmi la tyramine et l'octopamine.

6. Anticorps selon la revendication 1, caractérisés par le fait que ledit haptène est une indolamine.

7. Anticorps selon la revendication 6, caractérisés par le fait que ladite indolamine est choisie parmi la sérotonine, le 5-hydroxytryptophane, le 5-méthoxytryptophane, la 5-méthoxytryptamine, le tryptophane et la tryptamine

8. Anticorps selon la revendication 1, caractérisés par le fait que ledit haptène est un acide aminé.

9. Anticorps selon la revendication 8, caractérisés par le fait que ledit acide aminé est choisi parmi l'acide glutamique, l'acide aspartique, la taurine et l'acide gamma-aminobutyrique.

10. Anticorps selon la revendication 1, caractérisés par le fait que ledit haptène est un oligopeptide comportant 2 à 5 motifs d'acides aminés.

11. Anticorps selon l'une quelconque des revendications précédentes, caractérisés par le fait qu'ils sont marqués avec un traceur radioactif fluorescent ou enzymatique et/ou fixés sur un support.

12. Anticorps selon la revendication 1, caractérisés par le fait que ledit haptène est choisi parmi la tétrodotoxine, la saxitoxine et leurs analogues fonctionnels du type 2-imino hexahydropyrimidine.

13. Procédé de préparation des anticorps tels que définis dans

l'une quelconque des revendications précédentes, caractérisé par le fait que l'on immunise des animaux par administrations répétées, selon les méthodes connues, d'un ou plusieurs immunogènes de formule II :

$$[M]-[NH-(CH_2)_n-NH-Hapt.]_x \quad (II)$$

M étant le reste d'une macromolécule aminée de formule :

$$(NH=)_{x_2}[M]-(NH_2)_{x_1}$$

dans lequelle $-NH_2$ est un groupement amine primaire et =NH est l'imine d'un groupement du type guanidine, Hapt. étant le reste de la molécule d'haptène de formule Hapt.-$NH_2$ ou Hapt=NH, le groupement $-NH_2$ étant un groupement amine primaire et =NH étant l'imine d'un groupement de type guanidine, n étant un nombre entier pouvant varier de 4 et 6, $x_1$ étant un nombre égal ou supérieur à 1 et au plus égal au nombre de groupements amine primaire $-NH_2$ portés par ladite macromolécule aminée, et $x_2$ représente zéro ou un nombre au plus égal au nombre de groupements de type guanidine portés par ladite macromolécule aminée, (avec $x_1+x_2 = x$), puis que l'on recueille les anticorps sériques desdits animaux immunisés et que, si désiré, on fixe lesdits anticorps sur un support selon les méthodes connues et/ou les fait réagir avec un marqueur radioactif, fluorescent ou enzymatique selon les méthodes connues.

14. Nouveaux antigènes constitués par un haptène, comportant un groupement amine primaire ou un groupement de type guanidine, ledit haptène étant lié à une molécule aminée au moyen d'un agent de couplage, caractérisé par le fait qu'ils répondent à la formule III :

$$[M']-[NH-(CH_2)_n-NH-Hapt.]_x \quad (III)$$

M' étant le reste d'une molécule aminée de formule :

$$(HN=)_{x_2}[M']-(NH_2)_{x_3}$$

Hapt. étant le reste de la molécule d'haptène de formule Hapt.-$NH_2$ ou Hapt. = NH, les groupements $-NH_2$ étant des groupements amine primaire et les groupements = NH étant des imines de groupements de type guanidine,

n étant un nombre entier pouvant varier de 4 à 6,

$x_3$ étant un nombre, éventuellement nul, au plus égal au nombre de groupements amine primaire $-NH_2$ portés par ladite molécule aminée, et

$x_2$ étant zéro ou un nombre au plus égal au nombre de groupements de type guanidine portés par ladite molécule aminée, (avec $x_3+x_2 = x$).

15. Antigènes selon la revendication 14, caractérisés par le fait que ladite molécule aminée contient au moins un motif de lysine à groupement $\varepsilon$-$NH_2$ libre.

16. Antigènes selon l'une quelconque des revendications 14 et 15, caractérisés par le fait que ladite molécule aminée est une macromolécule antigénique.

17. Antigènes selon l'une quelconque des revendications 14 à 16, caractérisés par le fait que ladite molécule aminée est une protéine, un fragment de protéine, ou un polypeptide synthétique ou semi-synthétique.

18. Procédé de préparation d'un antigène tel que défini dans l'une quelconque des revendications 14 à 17, caractérisé par le fait :

a) que l'on fait réagir un dialdéhyde de formule :

$$O = CH - (CH_2)_{n-2} - CH = O$$

sur une solution de l'haptène et sur une solution de la molécule aminée,

b) et que l'on soumet le produit obtenu, de formule V :

$$\left[ Hapt - Y - (CH_2)_{n-2} - CH = \overset{+}{N} \right]_{x_2} \left[ M' \right] \left[ N = CH - (CH_2)_{n-2} - Y - Hapt. \right]_{x_3}$$

dans laquelle $x_3$ et $x_2$ sont définis comme précédemment, Y représente un groupement $- N = CH-$ lié par l'azote au reste Hapt lorsque l'haptène a réagi par sa fonction amine primaire, et Y représente un groupement $= \overset{\oplus}{N} = CH-$ lié par l'azote au reste Hapt. lorsque l'haptène a réagi par son groupement de type guanidine,
à l'action d'un agent réducteur capable de réduire les groupements imine ou les groupements $= \overset{\oplus}{N} = CH-$ en groupement amine.

19. Procédé selon la revendication 18, caractérisé par le fait que l'on fait réagir le dialdéhyde soit avec une solution contenant l'haptène et la molécule aminée, soit avec la molécule aminée puis avec l'haptène, soit avec l'haptène puis avec la molécule aminée.

20. Procédé selon l'une quelconque des revendications 18 et 19, caractérisé par le fait que l'on ajoute le dialdéhyde en excès.

21. Procédé selon l'une quelconque des revendications 18 à 20, caractérisé par le fait que ledit agent réducteur est un hydrure.

22. Application des anticorps tels que définis dans l'une quelconque des revendications 1 à 12, caractérisée par le fait que, pour détecter ou doser un haptène en solution ou sur une coupe de tissu cellulaire, on fixe l'haptène soit en mettant en contact un support revêtu d'une molécule aminée avec la solution à tester additionnée d'un dialdéhyde, soit en mettant en contact la coupe de tissu avec une solution de dialdéhyde, ledit dialdéhyde étant identique à celui qui a servi à préparer l'immunogène utilisé pour obtenir les anticorps, puis, après réduction éventuelle des liaisons imine néoformées, on met en contact le support ou le tissu fixé avec une solution contenant les anticorps marqués capables de reconnaître sélectivement l'haptène étudié.